# EUROPÄISCHE PATENTANMELDUNG

(11) **EP 2 463 327 A2**
(43) Veröffentlichungstag der Anmeldung: **13.06.2012**
(21) Anmeldenummer: 11191841.3
(22) Anmeldetag: 05.12.2011
(51) Int. Cl.: C08J 3/12, A61K 9/16

(54) **Verfahren zur herstellung von granulaten, enthaltend mindestens eine wasserlösliche komponente**

(30) Priorität: 10.12.2010 EP 10194454
(71) Anmelder: BASF SE, 67056 Ludwigshafen (DE)
(72) Erfinder: Maschke, Angelika, 68159 Mannheim (DE); Kolter, Karl, 67117 Limburgerhof (DE); Güntherberg, Norbert, 67346 Speyer (DE)

(57) **Zusammenfassung**

Verfahren zur Herstellung von Granulaten auf Basis mindestens eines thermoplastisch verarbeitbaren Polymeren, wobei die Granulate mindestens eine wasserlösliche Komponente enthalten, durch Schmelzextrusion und Formgebung in einem Kühlmedium, dadurch gekennzeichnet ist, dass als Kühlmedium ein Nichtlösemittel für das Polymer eingesetzt wird, wobei das Kühlmedium eine kinematische Viskosität von < 20 mm²/s, gemessen bei 40 °C, aufweist.

## Beschreibung

Die vorliegende Erfindung betrifft ein Verfahren zur Herstellung von Granulaten auf Basis thermoplastisch verarbeitbarer Polymere, wobei die Granulate mindestens eine wasserlösliche Komponente enthalten, durch Schmelzextrusion und Granulation in einem Kühlmedium sowie entsprechende Granulate und deren Verwendung.

Um die Handhabung von Polymeren bei den auf die Herstellung folgenden Verarbeitungsschritten zu vereinfachen, werden diese häufig in ein körniges und rieselfähiges und damit leicht förderbares Granulat überführt. Dieses geschieht, indem das geschmolzene Polymer durch eine Lochplatte gedrückt und danach in einem Wasserbad abgekühlt wird. Der so gebildete verfestigte Polymerstrang wird mittels einer rotierenden Messerwalze auf wenige mm lange, mehr oder wenige zylinderförmige Granulatkörner geschnitten, die nach Trocknen des oberflächlich anhaftenden Wassers verpackt und/oder der Weiterverarbeitung zugeführt werden. Eine andere häufig benutzte Variante ist die so genannte Unterwassergranulierung, bei der die Polymerschmelze durch eine Düsenplatte gedrückt wird, auf deren produktabgewandter Seite in einer wassergefluteten Schneidkammer ein rotierendes meist mehrschneidiges Messer die durch die Düsenplatte durchtretende Polymerschmelze zu kleinen Portionen abtrennt, die von dem die Kammer durchströmenden Kühlwasser wegtransportiert werden und dabei zu kugel- oder linsenförmigen Granulat erstarren. Diese Verfahren werden angewandt für Polymere, die sich nicht im Wasser lösen.

Entsprechende Vorrichtungen und Verfahren sind beispielsweise in der DE-A 2646309, der US-A 4264553 oder der US-A 5143673 beschrieben. Hierbei wird die heiße plastische Schmelze aus einem Extruder durch eine Lochscheibe direkt in ein flüssiges Kühlmedium, in diesem Fall Wasser, gefördert. Die austretenden Plastikstränge werden durch eine an der Lochscheibe angebrachten Schneidvorrichtung in Granulate zerteilt und durch das meist im Kreislauf gefahrene Wasser wegtransportiert, abgetrennt und getrocknet. Durch diesen, flüssigkeitsgekühlten Prozess ist es möglich sehr kleine Partikelgrößen einheitlich, kontinuierlich und im technischen Maßstab bis herunter zum Submillimeterbereich zu produzieren. Durch die Verwendung von Kühlmedien mit einer hohen Wärmekapazität und einem hohen Wärmeübergang wie Wasser, werden die noch plastischen Granulate schnell abgekühlt, verkleben nicht und lassen sich in Form und Größe sehr einheitlich herstellen.

Die Granulierung von wasserlöslichen oder wasserquellbaren Polymeren ist auf diesem Wege problematisch, da das Polymer sich beim Abkühlen im Wasser ganz oder teilweise lösen kann, oder dass der Strang oder das Granulat oberflächlich angelöst werden und miteinander verkleben, was den Prozess zum erliegen bringen kann.

Gängige Verfahren zum Granulieren von wasserlöslichen Polymeren sind das Abkühlen unter Luft oder Schutzgas, wobei der aus dem Extruder austretende Polymerstrang zum Abkühlen auf einem Band abgelegt und dann granuliert wird. Hierfür geeignet sind Teflon- oder Kettenbänder, oder gekühlte Stahlbänder (Sandvik-Band), die in gleicher Geschwindigkeit wie der Abgezogene Strang mitlaufen. Erfahrungsgemäß ist die Kapazität derartiger Verfahrensvarianten im Verhältnis zu den wassergekühlten Verfahren sehr gering, was die auf diesem Wege hergestellten Produkte teuer macht.

Eine andere Variante ist der Heißabschlag, bei dem die heiße Polymerschmelze durch rotierende Messer beim Austritt aus dem Extruder abgeschlagen und durch einen Luftstrom gekühlt werden. Dieses Verfahren ist jedoch nicht für jedes Polymer geeignet und in der Durchsatzkapazität wegen der geringen Kühlkapazität der Luft eingeschränkt.

Wirkstoffhaltige Formulierungen, die beispielsweise als Wirkstoff Vitamine enthalten, werden zur Gesunderhaltung von Mensch und Tier parallel zur Nahrungsaufnahme verabreicht oder der Nahrung als Additiv zugesetzt. Ein großer Anteil der hergestellten Formulierungen von Vitaminen, Vitaminoiden oder anderen Nahrungsergänzungsmitteln wird für die Tierernährung benötigt. Da die dort verwendeten Futtermittel als Mahlgut mit einer mittleren Teilchengröße von 0.3-0.5mm verabreicht werden, sollten zugesetzte Futtermitteladditive um Entmischungen zu vermeiden etwa die gleiche Größe und Einheitlichkeit aufweisen. Auch bei der Herstellung von Granulaten für Arzneimittel ist eine einheitliche Korngröße von besonderer Bedeutung, da das Auflösungsverhalten und damit die Bioverfügbarkeit von der Korngröße abhängen. Die bisher verwendeten Arzneimittelgranulate, Nahrungsergänzungsmittel oder Futtermitteladditive werden demzufolge meist in aufwendigen und teuren Mahl-, Granulier- und Sprühverfahren hergestellt.

Wirkstoffhaltige Zubereitungen, die durch Schmelzextrusion hergestellt werden, sind allgemein bekannt. Das Extrudieren von wirkstoffhaltigen Schmelzen wasserlöslicher Polymere, vorzugsweise von Copolymeren des Vinylpyrrolidons, ist beispielsweise aus der EP-A 240904 und der EP-A 240906 bekannt. In der EP-A 240 906 ist zudem die Schmelzextrusion von wirkstoffhaltigen Mischungen, die Copolymerisate von Methylmethacrylat und Acrylsäure, Copolymerisate von Vinylacetat und Crotonsäure als auch Ethylen/Vinylacetat-Copolymerisate beschrieben. Die Formgebung erfolgt über Spritzguß oder Extrusion mit anschließender Verformung des noch plastischen Stranges, z.B. durch Heißabschlag zu Granulat oder Verformen zu Tabletten. Die Formgebung erfolgt in allen genannten Beispielen an der Luft. Die so erzeugten pharmazeutischen Formen sind im Allgemeinen wasserlöslich.

So beschreibt zum Beispiel auch die DE-A 19536387 die Schmelzextrusion und Formgebung vitaminhaltiger Produkte. Als Matrix werden wasserlösliche, thermoplastische Hydroxypropylcellulosen verwendet. In den Beispielen werden unter anderem Schmelzen aus Vitamin C oder ß-Carotin zusammen mit Hydroxypropylcellulose durch Formkalander zu Tabletten gepresst. Zudem wird der Heißabschlag der wasserlöslichen Matrix zu Pellets erwähnt.

Zwar kann man solche wasserlöslichen Formulierungen mit Hilfe der Schmelzextrusion und anschließender Granulierung an der Luft herstellen. Nachteilig ist aber, dass man auf diese Art häufig aufgrund der schlechten Wärmeabführung nicht die erforderliche (kleine) Teilchengröße, Einheitlichkeit und großtechnische Realisierbarkeit erreichen kann.

Aus der US 6,632,389 ist die Herstellung von Pellets, enthaltend biologisch aktive Substanzen, durch Unterwassergranulation oder Granulation unter Kohlenwasserstoffen bekannt. Ausführlich beschrieben ist die Unterwassergranulation von pH-abhängig löslichen Polymeren durch entsprechende Kontrolle des pH-Werts im Kühlmedium. Die Granulation in Kühlmedien wie Mineralölen oder Pflanzenölen wird nur ganz allgemein erwähnt.

Allerdings hat sich gezeigt, dass Kohlenwasserstoffe nicht grundsätzlich als Kühlmedium geeignet sind.

Aufgabe der Erfindung war es, ein verbessertes Verfahren zur Granulierung von thermoplastisch verarbeitbaren wasserlöslichen oder wasserquellbaren Polymeren in einem Kühlmedium zu finden.

Demgemäß wurde ein Verfahren zur Herstellung von Granulaten auf Basis mindestens eines thermoplastisch verarbeitbaren Polymeren, wobei die Granulate mindestens eine wasserlösliche Komponente enthalten, durch Schmelzextrusion und Formgebung in einem Kühlmedium , gefunden, welches dadurch gekennzeichnet ist, dass als Kühlmedium ein Nichtlösemittel für das Polymer eingesetzt wird, wobei das Kühlmedium eine Viskosität von < 20 mm²/s, gemessen bei 40 °C, aufweist.

Vorzugsweise eignet sich das erfindungsgemäße Verfahren zur Herstellung von Granulaten, in denen mindestens eine biologisch aktive Substanz (Wirkstoff) homogen dispergiert in einer Matrix des Polymers vorliegt. Dabei kann der Wirkstoff im Polymer gelöst sein (feste Lösung) oder dispergiert sein. Dispergiert bedeutet, dass der Wirkstoff molekulardispers (< 1 nm), kolloidaldispers (1- 500 nm), grobdispers (< 500 nm), mikroskopisch grobdispers/feindispers (500 nm bis 100 µm) oder makroskopisch grobdispers (> 100 µm) vorliegt, wobei die Zahlenangaben in der Klammer sich auf die mitteleren Teilchengrößen der Wirkstoffphase beziehen.

Molekulardisperse Systeme werden auch als "feste Lösungen" bezeichnet. Feste Lösungen sind röntgenamorph, was bedeutet, dass im Röntgendiffraktogramm weniger als 5 Gew.-% kristalline Anteile zu finden sind. Der Begriff "feste Lösungen" ist dem Fachmann geläufig (s. Chiou und Riegelman, J. Pharm. Sci. 60, 1281-1302 (1971)).

Gemäß einer Ausführungsform der Erfindung handelt es sich bei der wasserlöslichen Komponente um die biologisch aktive Substanz.

Gemäß einer anderen Ausführungsform der Erfindung handelt es sich bei der wasserlöslichen Komponente um ein wasserlösliches, thermoplastisch verarbeitbares, Polymer.

Gemäß einer weiteren Ausführungsform der Erfindung enthalten die Granulate eine wasserlösliche biologisch aktive Substanz und mindestens ein wasserlösliches thermoplastisch verarbeitbares Polymer.

Eine weitere Ausführungsform der Erfindung betrifft Granulate, die als Matrixpolymere Gemische von mindestens einem wasserlöslichen Polymer mit in Wasser schwerlöslichen oder unlöslichen Polymeren enthalten.

Insbesondere wurde ein Verfahren zur Herstellung von Granulaten, in denen biologisch aktiven Substanzen homogen dispergiert in einer Matrix auf Basis mindestens eines thermoplastisch verarbeitbaren Polymeren, vorliegen, wobei die Granulate durch homogenes Vermischen der Einsatzstoffe in der Schmelze und anschließender Extrusion und Formgebung erhalten werden, gefunden.

Grundsätzlich eignen sich für alle Ausführungsformen Polymere, die als solche thermoplastisch verarbeitbar sind oder in einer Mischung mit anderen Polymeren und/oder anderen Hilfsstoffen thermoplastisch verarbeitbar sind.

Je nach Ausführungsform der Erfindung können wasserlösliche, pH-abhängig lösliche, schwerlösliche oder unlösliche Polymere als Matrixpolymere eingesetzt werden. Als wasserlösliche thermoplastisch verarbeitbare Polymere können auch Polymere eingesetzt werden, die eine LCST (Lower Critical Solution Temperature) aufweisen. Bezüglich der Löslichkeit der Polymere sind die Begriffe "wasserlöslich" und "in Wasser schwerlöslich" wie folgt zu verstehen: Der Begriff "wasserlöslich" bedeutet, dass für eine Lösung des Polymers in Wasser bei 20 °C 1 bis 30 g Wasser pro g Polymer benötigt werden .Der Begriff "in Wasser schwerlöslich" umfasst erfindungsgemäß wenig lösliche, schwerlösliche sowie auch praktisch unlösliche Substanzen und bedeutet, dass für eine Lösung des Polymers in Wasser bei 20 °C von 30 g bis 1000 g Wasser pro g Polymer benötigt werden Bei praktisch unlöslichen Substanzen werden mindestens 10.000 g Wasser pro g Substanz benötigt. Die erfindungsgemäß als wasserlösliche Polymere bezeichneten Substanzen sind über den gesamten pH-Bereich wasserlöslich.

In der folgenden Beschreibung wird der Begriff "schwerlöslich" verkürzt für "in Wasser schwerlöslich" benutzt. Unter den schwerlöslichen Polymeren werden auch solche Polymere aufgeführt, die nicht über den vollen pH-Bereich löslich sind, sondern eine pHabhängige Löslichkeit zeigen.

Geeignete wasserlösliche Polymere sind beispielsweise wasserlösliche Homo- und Copolymere des N-Vinylpyrrolidons. So eignen sich Homopolymere mit K-Werten nach Fikentscher von 10 bis 100, insbesondere K12, K15, K30 K 60, K90. Bei den Copolymeren des N-Vinylpyrrolidons eignen sich vor allem Copolymere mit Vinylacetat, bevorzugt solche mit einem Gewichtsverhältnis von N-Vinylpyrrolidon (NVP) zu Vinylacetat (VAc) 60: 40 bis 80:20, insbesondere das nach der Pharm Eur. und der US-Pharmakopöe als Copovidon bezeichnete Copolymer aus NVP/ VAc im Gewichtsverhältnis 60:40

Weiterhin eignen sich als wasserlösliche polymere Polyvinylalkohole, die als Homopolymere üblicherweise durch Hydrolyse von Polyvinylacetat erhalten werden.

Ebenso eignen sich Pfropfpolymere auf Basis von Polyethern als Pfropfgrundlage. Solche Pfropfcopolymere können durch radikalische Polymerisation von Vinylmonomeren in Gegenwart Polyethern erhalten werden. Als Vinylmonomere eignen sich beispielsweise N-Vinyllactam-Monomeren wie N-Vinylpyrrolidon oder N-Vinylcaprolactam oder Mischungen davon. Weiterhin eignen sich als Vinylmonomere Vinylether oder Vinylester, insbesondere Vinylacetat. Im Falle von Pfropfcopolymeren, die unter Verwendung von Vinylacetat erhalten werden, können die Estergruppen auch teilweise oder vollständig hydrolysiert vorliegen. Weiterhin eignen sich auch Acrylate oder Methacrylate als Comonomere. Bevorzugt eignen sich Polyvinylalkohol-Polyethylenglykol-Pfropfcopolymere oder Pfropf-Copolymere, die durch radikalische Polymerisation einer Mischung aus Polyethylenglykol, N-Vinylcaprolactam und/oder Vinylacetat erhalten werden können. Solche Pfropfpolymere sind kommerziell als Kollicoat® IR oder Soluplus®, Firma BASF, erhältlich.

Weiterhin kommen als wasserlösliche Polymere Polyalkylenglykole wie z.B. Polyethylenglykole, Ethylenglykol-Propylenglykol-Blockcopolymere, Hydroxyalkylierte Cellulosen wie z.B. Hydroxypropylmethylcellulose, Hydroxypropylcellulose, Hydroxyethylcellulose, Polysaccharide wie z.B. Carragenane, Pektine, Xanthane oder Alginate in Betracht.

In Wasser schwerlösliche Polymere im Sinne der Erfindung sind entweder neutrale schwerlösliche Polymere (Retardpolymere), anionische schwerlösliche Polymere (magensaftresistente Polymere) oder basische schwerlösliche Polymere

Unter neutralen schwerlöslichen Polymeren werden solche Polymere verstanden, die über den gesamten pH-Bereich von 1 bis 14 wasserschwerlöslich oder lediglich in Wasser quellbar sind. In der Regel ist in der pharmazeutischen Zusammensetzung nur ein wasserunlösliches Polymer enthalten. Es können jedoch gegebenenfalls auch zwei oder mehr wasserunlösliche Polymere nebeneinander oder in Mischung vorliegen. Geeignete schwerlösliche Polymere sind beispielsweise: Neutrale oder im Wesentlichen neutrale Methacrylat-Copolymere. Diese können insbesondere aus mindestens 95, insbesondere mindestens 98, bevorzugt mindestens 99, insbesondere zu mindestens 99, besonders bevorzugt zu 100 Gew.-% aus radikalisch polymerisierten (Meth)acrylatMonomeren mit neutralen Resten, insbesondere C1- bis C4-Alkylresten, bestehen.

Geeignete (Meth)acrylat-Monomere mit neutralen Resten sind z. B. Methylmethacrylat, Ethylmethacrylat, Butylmethacrylat, Methylacrylat, Ethylacrylat, Butylacrylat. Bevorzugt sind Methylmethacrylat, Ethylacrylat und Methylacrylat. In geringen Anteilen, zu weniger als 5, bevorzugt höchstens 2, besonders bevorzugt höchstens 1 oder 0,05 bis 1 Gew.-% können Methacrylatmonomere mit anionischen Resten, z. B. Acrylsäure und/oder Methacrylsäure, enthalten sein. Geeignet sind z. B. neutrale oder nahezu neutrale (Meth)acrylat Copolymere aus 20 bis 40 Gew.-% Ethylacrylat, 60 bis 80 Gew.-% Methylmethacrylat und 0 bis weniger als 5, bevorzugt 0 bis 2 oder 0,05 bis 1 Gew.-% (Typ Eudragit® N E). Eudragit N E ist ein Copolymer aus 30 Gew.-% Ethylacrylat und 70 Gew.-% Methylmethacrylat.

Weitere geeignete schwerlösliche (Meth)acrylat-Copolymere sind beispielsweise unabhängig vom pH-Wert lösliche oder quellbare Polymerisate, die für Arzneimittelüberzüge geeignet sind.

Das schwerlösliche Polymer kann ein Polymerisat aus 98 bis 85 Gew.-% C1- bis C4-Al kylestern der Acryl- oder der Methacrylsäure und 2 bis 15 Gew.-% (Meth)acrylat Monomeren mit einer quaternären Ammoniumgruppe ist oder eine Mischung mehrerer Polymer dieser Substanzklasse sein.

Das schwerlösliche Polymer kann auch ein Polymerisat aus 97 bis mehr als 93 Gew.-% C1- bis C4-Alkylestern der Acryl- oder der Methacrylsäure und 3 bisweniger als 7 Gew.-% (Meth)acrylat Monomeren mit einer quaternären Ammoniumgruppe sein (Typ Eudragit® RS). Bevorzugte C1- bis C4-Alkylester der Acryl- oder der Methacrylsäure sind Methylacrylat, Ethylacrylat, Butylacrylat, Butylmethacrylat und Methylmethacrylat. Als (Meth)acrylat Monomer mit quaternären Aminogruppen wird 2-Trimethylammoniumethylmethacrylat-Chlor besonders bevorzugt. Ein beispielhaft geeignetes Copolymer enthält 65 Gew.-% Methylmethacrylat, 30 Gew.-% Ethylacrylat und 5 Gew.-% 2-Trimethylammonium-ethylmethacrylat Chlorid (Eudragit RS).-

Das schwerlösliche Polymer kann ein Polymerisat aus 93 bis 88 Gew.-% C1- bis C4-Alkylestern der Acryl- oder der Methacrylsäure und 7 bis 12 Gew.-% (Meth)acrylatMonomeren mit einer quaternären Ammoniumgruppe sein (Typ Eudragit RL). Ein konkret geeignetes Copolymer enthält z. B. 60 Gew.-% Methylmethacrylat, 30 Gew.-% Ethylacrylat und 10 Gew.-% 2-Trimethyl-ammoniumethlymethacrylat-Chlorid (Eudragit® RL).

Das wasserunlösliche Polymer kann eine Mischung der Polymere vom Typ Eudragit RS und vom Typ Eudragit RL im Verhältnis 20 zu 1 bis 1 zu 20 sein. Insbesondere geeignet sind auch Mischungen aus EUDRAGIT RS und Eudragit RL z. B. im Verhältnis von 20 : 1 bis 1 : 20 Gewichtsteilen.

Die pharmazeutische Zusammensetzung kann als schwerlösliches Polymer auch ein Polyvinylacetat enthalten. Als Polyvinylacetate eignen sich beispielsweise die Homopolymerisate des Vinylacetats. Weiterhin eignen sich schwerlösliche Polyvinylacetat-Copolymere, beispielsweise wasserunlösliche Copolymere aus Vinylacetat und N-Vinylpyrrolidon. Kommerziell erhältliche geeignete Polyvinylacetate sind beispielsweise Kollicoat® SR 30D oder Kollidon® SR.

Als schwerlösliche Polymere eignen sich auch Alkylcellulosen wie beispielsweise Ethylcellulose und neutrale Celluloseester wie z.B. Celluloseacetatbutyrat.

Weiterhin können auch anionische schwerlösliche Polymere verwendet werden. Unter anionischen Polymeren werden bevorzugt Polymere mit mindestens 5 %, besonders bevorzugt 5 bis 75 % Monomerresten mit anionischen Gruppen verstanden. Bevorzugt sind anionische (Meth)acrylat-Copolymere. Geeignete kommerziell erhältiche (Meth)acrylatcopolymere mit anionischen Gruppen sind beispielsweise die Eudragit®-Typen L, L100-55, S und FS.

Geeignete anionische (Meth)acrylatcopolymere sind z. B. Polymerisate aus 25 bis 95, Gew.-% C1- bis C4-Alkylestern der Acryl- oder der Methacrylsäure und 5 bis 75 Gew.-% (Meth)acrylat-Monomeren mit einer anionischen Gruppe. Entsprechende Polymere sind je nach Gehalt an anionischen Gruppen und dem Charakter der weiteren Monomere bei pH-Werten oberhalb von pH 5,O wasserlöslich und somit auch darmsaftlöslich. In der Regel addieren sich die genannten Anteile zu 100 Gew.-%.

Ein (Meth)acrylat-Monomer mit einer anionischen Gruppe kann z. B. Acrylsäure, bevorzugt jedoch Methacrylsäure sein. Weiterhin geeignet sind anionische (Meth)acrylat Copolymere aus 40 bis 60, Gew.-% Methacrylsäure und 60 bis 40 Gew.-% Methylmethacrylat oder 60 bis 40 Gew.-% Ethylacrylat. (Typen Eudragit L oder Eudragit L1 00-55). EUDRAGIT L ist ein Copolymer aus 50 Gew.-% Methylmethacrylat und 50 Gew.-% Methacrylsäure.

Eudragit L1 00-55 ist ein Copolymer aus 50 Gew.-% Ethylacrylat und 50 Gew.-% Methacrylsäure. Eudragit L 30D-55 ist eine Dispersion enthaltend 30 Gew.-% Eudragit L 100-55.

Ebenso geeignet sind anionische (Meth)acrylat Copolymere aus 20 bis 40 Gew.-% Methacrylsäure und 80 bis 60 Gew.-% Methylmethacrylat (Typ Eudragit® S). Geeignet sind weiterhin z. B. anionische (Meth)acrylat Copolymere, bestehend aus 10 bis 30 Gew.-%, Methylmethacrylat, 50 bis 70 Gew.-% Methylacrylat und 5 bis 15 Gew.-% Methacrylsäure (Typ Eudragit® FS).

Eudragit FS ist ein Copolymer aus 25 Gew.-%, Methylmethacrylat, 65 Gew.-% Methylacrylat und 10 Gew.-% Methacrylsäure. Eudragit FS 30 D ist eine Dispersion enthaltend 30 Gew.-% Eudragit® FS. Die Copolymere bestehen bevorzugt im Wesentlichen bis ausschließlich aus den Monomeren Methacrylsäure, Methylacrylat und Ethylacrylat in den oben angegebenen Mengenanteilen.

Es können jedoch zusätzlich, ohne dass dies zu einer Beeinträchtigung der wesentlichen Eigenschaften führt, geringe Mengen im Bereich von 0 bis 10, z. B. 1 bis 5 Gew.-% weiterer vinylisch copolymerisierbarer Monomere, wie z. B. Methylmethacrylat, Butylmethacrylat, Butylacrylat oder Hydroxyethylmethacrylat enthalten sein.

Partielle Ester der Cellulose oder hydroxyalkylierter Cellulose mit mehrwertigen Säuren wir Bernsteinsäure, Phthalsäure, Trimellitinsäure, wie z. B. Celluloseacetatphthalat, Celluloseacetatsuccinat, Celluloseacetattrimellitat, Hydroxypropylcelluloseacetatsuccinat, Hydroxypropylcelluloseacetatphtalat,

Basische schwerlösliche Polymere: Es können auch basische Polymere wie basische Meth(acrylate) oder Chitosan eingesetzt werden. Ein Beispiel für ein entsprechendes kommerziell erhältliches Polymer ist Eudragit® E oder EPO, das ein Copolymer aus Methylmethacrylat, Butylmethacrylat und Dimethylaminoethylmethacrylat darstellt.

Weiterhin können auch thermoplastische Zuckeralkohole wie z.B. Isomalt (kommerziell als Palatinit® erhältlich) eingesetzt werden.

Gemäß einer bevorzugten Ausführungsform eignen sich Mischungen aus wasserlöslichen und nicht wasserlöslichen Polymeren. So eignen sich beispielsweise Mischungen aus Copovidon und Kollidon® SR (Firma BASF SE). Kollidon SR ist eine kommerziell erhältliche co-prozessierte Mischung aus 80 Gew.-% Polyvinylacetat, 19 Gew.-% Povidon (Kollidon 30), 0.8 gew.-% Natrium-Laurylsulfat und 0.2 Gew.-% Silica.

Als Kühlmedium eignen sich Fluide mit einer kinematischen Viskosität von < 20 mm²/s, gemessen bei 40 °C nach DIN 51562. Grundsätzlich geeignet sind Fluide im Bereich von 0,1 mm²/sec bis kleiner als 20 mm²/sec. Hierbei ist zu beachten, dass Fluide im Bereich von 0.1 mm²/sec bis 2 mm²/sec aufgrund des relativ hohen Dampfdruckes (niedrigen Siedepunktes) an der Luft relativ leicht zündfähige Gemische bilden und nur unter speziellen Explosionsschutz-Massnahmen gehandhabt werden können, andererseits aber sehr leicht durch Verdampfen von der Oberfläche entfernt werden können. Gemäß einer Ausführungsform der Erfindung, werden Fluide mit kinematischen Viskositäten im Bereich von 10 mm²/s bis 19 mm²/s, bevorzugt 18 bis 11, besonders bevorzugt 17 bis 12 mm²/s eingesetzt. Diese bevorzugte Ausführungsform ermöglicht Arbeiten ohne erhöhte aufwändige Explosionsschutz-Massnahmen.

Während der Granulierung wird das Kühlmedium auf 10 bis 90 °C, vorzugsweise 15 bis 80 °C, besonders bevorzugt 20 bis 50 °C temperiert.

Der zu wählende Bereich hängt von den Schmelz- beziehungsweise Erweichungspunkten der eingesetzten Polymeren und Wirkstoffsysteme ab. Im Allgemeinen wählt man eine Temperatur, die genügend weit unter dem Erweichungs/Schmelzpunkt des Systems liegt, um ein sauberes Erstarren bei Abkühlung zu ermöglichen. Die Badtemperatur kann auf Temperaturen im Bereich von 50, 70 oder 100 °C unter dem Schmelzpunkt eingestellt werden. Welche Temperatur im Einzelfall am Besten geeignet ist, kann vom Fachmann auf einfache Weise ermittelt und ausgewählt werden.

Zur Durchführung des erfindungsgemäßen Verfahrens werden die Polymere oder eine Mischung aus Polymeren und weiteren Komponenten zu einer homogenen Schmelze vermischt. Dies kann beispielsweise erfolgen, indem man ein physikalische Vormischung fester Einsatzstoffe in einen geeigneten Extruder oder Kneter einbringt, die Mischung unter Einsatz von mechanischer und thermischer Energie aufschmilzt, und die heiße, noch plastische Schmelze durch eine Lochscheibe oder eine Düsenplatte direkt in das Kühlmedium fördert. Enthält die Formulierung auch flüssige Inhaltsstoffe, so empfiehlt es sich, diese getrennt über eine Dosierpumpe zuzugeben. Verwendet man thermisch labile Wirkstoffe, so kann es sich auch empfehlen, zunächst eine Schmelze der thermoplastisch verarbeitbaren Polymere und gewünschtenfalls weiterer Formulierungshilfsstoffe herzustellen, und dann erst den Wirkstoff zuzugeben. Das Aufschmelzen erfolgt vorzugsweise in einer Schneckenmaschine, insbesondere in einem Doppelschneckenextruder, der vorzugsweise gleichsinnig drehend ist. Das erfindungsgemäße Verfahren erfolgt vorzugsweise in Abwesenheit von Lösungsmitteln. Im Falle, dass es erforderlich sein sollte, einen oder mehreren der Einsatzstoffe in Form einer Lösung zu der Mischung zu geben, können die dabei verwendeten Lösungsmittel in den hinteren Extruderzonen durch einfaches Verdampfen, gegebenenfalls unter Anlegen eines Vakuums entfernt werden. Die extrudierte, noch thermoplastische Masse enthält dann kein Lösungsmittel mehr.

Das Aufschmelzen der Einsatzstoffe kann je nach Zusammensetzung der Mischung bei Temperaturen von 50 bis 300°C, vorzugsweise 70 bis 250 °C, erfolgen.

Die austretenden plastischen Stränge werden durch eine an der Lochscheibe oder Düsenplatte angebrachten Schneidvorrichtung in Granulate zerteilt. Als Granuliervorrichtungen eignen sich handelsübliche Vorrichtungen wie sie für die Unterwassgranulation eingesetzt werden. Dabei wird die polymerhaltige Schmelze von einem rotierenden Messer in der Messerkammer zu kleinen Tropfen zerteilt, die von dem fluiden Kühlmedium abtransportiert werden und zu mehr oder weniger runden kugel- oder linsenförmigen Teilchen erstarren.

Gemäß einer Ausführungsform der Erfindung werden diese Granulatkugeln mittels einer Zentrifuge vom Kühlmedium getrennt und ausgetragen. Für eine erfolgreiche Trennung mittels Zentrifuge ist es erforderlich, dass die Dichte des Kühlmediums geringer ist, als die Dichte der polymerhaltigen Granulate. Weiterhin empfiehlt es sich, die Maschenweite des Zentrifugensiebes auf die Viskosität des Kühlmediums und die angestrebte Korngröße der Granulate abzustimmen. Bevorzugt ist eine für die erwartete Korngröße möglichst große Maschenweite, da sonst eine unvollständige Trennung erfolgt, was zu Störungen des Verfahrens führt. Vorzugsweise beträgt bei dieser Ausführungsform das Verhältnis Maschenweite des Zentrifugensiebs zu mittlerer Korngröße 0,1 bis 0,95. Es können auch Zentrifugen mit Schlitzlochblechen verwendet werden, wobei in diesem Falle zahlenmässig das gleiche Verhältnis gewählt wird, das sich aber dann auf die Größe der Schmalseite der Schlitzöffnung bezieht.

Gemäß einer weiteren Ausführungsform kann die Trennung nicht mittels einer Zentrifuge, sondern durch Sieb und Filtersysteme durchgeführt werden. Dafür eignen sich beispielsweise Filterbeutel aus entsprechenden lichten Geweben, Kantenspaltfilter, Metallgewebefilter, Metallgewebeverbundplatten oder - Rohre, wobei die feinste Filterlage dem Produkt zugewandt ist. Diese Filtration kann unter Atmosphärendruck, Überdruck oder unter Vakuum erfolgen.

Als Kühlmedium wird ein fluides Medium eingesetzt, das für die wasserlösliche Komponenten als Nichtlösemittel wirkt. Unter Nichtlösemittel wird hier ein Fluid verstanden, das während der Kontaktzeit das wasserlösliche Polymer oder die wasserlösliche biologisch aktive Substanz der Granulate weder löst noch anquillt noch zum Verkleben bringt. Die Kontaktzeiten zwischen Kühlmedium und Granulaten betragen üblicherweise 0,02 bis 2 Minuten. Als derartige Fluide sind unpolare Fluide, wie beispielsweise lineare und verzweigte gesättigte und teilweise ungesättigte Kohlenwasserstoffe, Mineralölfraktionen, langkettige Alkohole und Fettsäuren und auch natürliche pflanzliche Öle wie Palmöl, Sonnenblumenöl, Olivenöl und dergleichen geeignet. Für gewisse Anwendungen kann auch Siliconöl eingesetzt werden. Die Auswahl des Nichtlösemittels richtet sich nach den zu granulierenden Polymeren und den Anforderungen an das Granulat (Einsatz im Lebensmittelbereich oder industrielle Verarbeitung, Entfernbarkeit der Fluidreste und dergleichen). Die hier genannten Fluide stellen lediglich Beispiele dar, ohne jedoch einschränkend zu sein. Wie bereits erwähnt weisen die gemäß der vorliegenden Erfindung als Kühlmedium geeigneten Fluide eine kinematische Viskosität bei 40 °C von kleiner 20 mm²/sec auf.

Die fluiden Kühlmedien werden vorzugsweise im Kreislauf gefahren.

Der Fluidkreislauf wird vorzugsweise gekühlt, um eine übermäßige Erwärmung auf höhere Temperaturen, insbesondere Temperaturen über der Glasübergangstemperatur der Polymere beziehungsweise die Erweichungstemperaturen des granulierten Stoffsystems, zu vermeiden. Vorzugsweise wird die Temperatur des Kühlmediums auf Temperaturen gehalten, die mindestens 50 °C unter der Schmelzetemperatur liegen.

Die auf diesem Wege hergestellten Granulate können noch geringe Mengen des Fluids enthalten, beispielsweise in Größenordnungen von 0.01-5 Gew.-% bezogen auf das Gesamtgewicht der Granulate, wobei die anhaftende Menge von der Korngröße der Granulate, den Eigenschaften des Kühlmediums und den eingestellten Verfahrensparametern in der Granuliervorrichtung abhängt. Üblicherweise muss das anhaftende Kühlmedium noch entfernt werden, sofern es nicht aufgrund des niedrigen Siedepunktes von selbst verdampft.

Dies kann mechanisch geschehen, beispielsweise mittels saugfähiger Medien wie Filz, Cellulose, Baumwollgewebe oder anderen Geweben oder durch Waschen, beispielsweise mit einem niedrig siedenden Kohlenwasserstoff, der das Kühlmedium gut löst und selbst schnell verdampft, ohne jedoch die Granulate anzulösen oder anzuquellen.

Mit Hilfe des erfindungsgemäßen Verfahrens lassen sich die Granulate in Form und Größe sehr einheitlich herstellen. Bevorzugt werden Granulate mit mittleren Teilchengrößen (Q50%) im Bereich von 0,05 bis 10, vorzugsweise von 0,1 bis 5 mm, insbesondere von 0,2 bis 3 mm hergestellt. Die Partikelgrößenverteilung wird als Summenverteilung analysiert: Q10% bedeutet, dass 10 % der Partikel kleiner als der angegebene Wert sind, Q50% bedeutet, dass 50 % kleiner als der angegebene Wert sind und Q90% bedeutet, dass 90 % kleiner als der angegebene Wert sind.

Die Bestimmung der Teilchengrößen kann mit einer Siebanalyse wie im Europäischen Arzneibuch angegeben erfolgen. Des Weiteren können optische Verfahren (Camsizer) eingesetzt werden.

Neben Polymeren und biologisch aktiven Substanzen können derartige Formulierungen übliche extrusionstechnische und formulierungsrelevante Zusätze, wie z.B. Copolymere, Weichmacher, Tenside, Stabilisatoren, farbgebende Mittel wie anorganische oder organische Pigmente, Aroma- und Süßungsmittel, Gleit- oder Trennmittel enthalten.

Als Weichmacher können z.B. Polyethylenglykole, Triacetin, Triethylcitrat oder Propandiol verwendet werden. Sofern Weichmacher verwendet werden, richtet sich deren Menge nach dem Gesamtverhalten der Einsatzstoffe. Die Menge an Weichmacher wird so eingestellt, dass die Schmelze extrudierbar und granulierbar ist. Die Mengen können je nach Charakter der Einsatzstoffe variieren.

Als Stabilisatoren eignen sich beispielsweise Antioxidantien oder Konservierungsmittel.

Als Tenside eignen sich grenzflächenaktive Substanzen mit einem HLB-Wert (HydrophilicLipophilicBalance) von 1 bis 40 (Vgl. Fiedler, Lexikon der Hilfsstoffe, Bd. 1, Seiten 77 - 82, 4. Auflage, Editio Cantor, Aulendor), beispielsweise mit 40 Ethylenoxid-Einheiten ethoxiliertes hydriertes Ricinusöl (Cremophor® RH 40), mit 35 Ethylenoxid-Einheiten ethoxiliertes Ricinusöl (Cremophor EL), Polysorbat 80, Poloxamere, Docusat-Natrium oder Natriumlaurylsulfat.

Je nach Anwendungsgebiet und Verarbeitbarkeit können die Anteile an Wirkstoff, Polymer und Additiven in weiten Grenzen variieren. Die einzigen Randbedingungen sind die thermoplastische Verarbeitbarkeit und die beschriebenen Löslichkeitseigenschaften der Formulierung. Im Allgemeinen wird der Wirkstoffgehalt in einem Bereich von 5 bis 90, vorzugsweise 5 bis 80, besonders bevorzugt 5 bis 60 Gew.-% liegen. Den Rest bilden Polymere, im Allgemeinen von 10 bis 55 Gew.-%, sowie Formulierungshilfsstoffe.

Das erfindungsgemäße Verfahren eignet sich prinzipiell zur Herstellung von Granulaten auf Basis thermoplastisch verarbeitbarerer Polymere, vorzugsweise zur Herstellung von biologisch aktive Substanzen enthaltenden Granulaten, in denen eine oder mehrere biologisch aktive Substanzen homogen in einer Matrix auf Basis thermoplastisch verarbeitbarer Polymere dispergiert vorliegen.

Als biologisch aktive Substanzen kommen generell alle Substanzen in Betracht, die im Magen-Darm-Trakt von Mensch und Tier freigesetzt werden sollen. Dies können beispielsweise Pharmawirkstoffe, Vitamine, Vitaminoide, Carotinoide, Enzyme, Hormone, Aminosäuren oder sogenannte "Nutraceuticals", also Nahrungsergänzungsmittel und dietätische Mittel, sein.

Zudem ist es möglich auch Pflanzenschutzwirkstoffe, Waschmittelinhaltsstoffe wie beispielsweise Enzyme, Geruchs- und Geschmacksstoffe oder andere Aktivsubstanzen in der oben beschriebenen Weise zu formulieren.

Das erfindungsgemäße Verfahren und die erfindungsgemäße Vorrichtung eignen sich zur Herstellung von partikulären Zubereitungen biologischer Substanzen. Biologisch aktive Substanzen sind erfindungsgemäß Stoffe, die in lebenden Organismen eine biologische Wirkung hervorrufen.

Das erfindungsgemäße Verfahren eignet sich beispielsweise zur Formulierung folgender Stoffe oder deren physiologisch akzeptablen Salzen, wobei die Salze auch in situ im Extruder erzeugt werden können:
Die verwendeten pharmazeutischen Wirkstoffe sind in Wasser lösliche, wenig lösliche, schwer lösliche oder unlösliche Substanzen. Gemäß DAB 9 (Deutsches Arzneimittelbuch) erfolgt die Einstufung der Löslichkeit pharmazeutischer Wirkstoffe wie folgt: (jeweils 1 Teil der Substanz ist in den jeweils genannten Mengen an Wasser löslich) sehr leicht löslich (löslich in 1 Teil Lösungsmittel), leicht löslich(löslich in 1 bis 10 Teilen Lösungsmittel), löslich (löslich in 10 bis 30 Teilen Lösungsmittel), wenig löslich (löslich in 30 bis 100 Teilen Lösungsmittel); schwer löslich (löslich in 100 bis 1000 Teilen Lösungsmittel); praktisch unlöslich (löslich in mehr als 10000 Teilen Lösungsmittel). Die Wirkstoffe können dabei aus jedem Indikationsbereich kommen.

Gemäß einer Ausführungsform der Erfindung eignet sich das erfindungsgemäße Verfahren für biologisch aktive Substanzen die gut wasserlöslich sind, was erfindungsgemäß bedeutet, dass sie sehr leicht löslich, leicht löslich oder löslich gemäß der oben angeführten Definition sind.
- Antiinfektiva
   Aciclovir, Aminoglykoside, Amphotericin B, Azol-Antimykotika, Clotrimazol, Itraconazol, Sepraconazol, Clindamycin, Cephalosporine, Chloramphenicol, Erythromycin, 5-Fluoruracil, Etoposid, Fluctytosin, Ganciclovir, Griseofulvin, Gyrasehemmstoffe, Isoniacid, Lincosamide, Mebendazol, Mefloquin, Metronidazol, Nitroimidazole, Novobiocin, Platinverbindungen, Polymyxin B, Praziquantel, Pyrimethamin, Rifamipicin, Saquinavir, Streptomycin, Sulfonamide, Tetracycline, Trimethoprim, Vancomycin, Zidovudin;
- Antipyretika, Analgetika, antiinflammatorische Mittel, Paracetamol, Ibuprofen, Ketoprofen, Oxaprozin, Acetylsalicylsäure, Morphin, Oxaprozin, Propoxyphen, Phenylbutazon;
- Antibiotika
   Rifampicin, Griseofulvin, Chloramphenicol, Cycloserin, Erythromycin, Penicilline wie Penicillin G, Streptomycin, Tetracyclin;
- Antiepileptika
   Hydantoine, Carbamazepin;
- Antitussiva und Antiasthmatika
   Diphenhydramin;
- Antirheumatika
   Chloroquin, Indomethacin, Goldverbindungen, Phenylbutazon, Oxyphenylbutazon, Penicillinamin;
- Hypnotika
   Barbiturate, Phenobarbital, Zolpidem, Dioxopiperidine, Ureide;
- Insektizide
   Aldrin, Dieldrin, Chlorphenotan, Hexachlorcyclohexan;
- Herbizide
   Vinclozolin, Strobilurine;
- Psychopharmaka, Neuroleptika
   Perazin, Promazin, Sulpirid, Thioridazin, Chlorpromazin, Meprobamat, Triflupromazin, Melperon, Clozapin, Risperdion, Reserpin;
- Tranquillantien;
- Antidepressiva
   Imipramin, Paroxetin, Viloxazin, Moclobemid;
- Psychotonika;
- Psychomimetika;
- Diuretika
   Kaliumcanrenoat, Schleifendiuretika, Furosemid, Hydrochlorothiazid, Spironolacton, Thiazide, Triamteren;
- Hormone
   Androgene, Antiandrogene, Gestagene, Glucocorticoide, Oestrogene, Cortisol, Dexamethason, Prednisolon, Testosteron, Adiuretin, Oxytocin, Somatropin, Insulin;
- Immunsuppresiva
   Ciclosporin;
- Bronchodilatoren;
- Muskelrelaxantien, Tranquillantien
   Carisoprodol, Tetrazepam, Diazepam, Chlordiazepoxid;
- Enzyme
   Lipase, Phytase;
- Gichtmittel
   Allopurinol, Colchicin;
- Antikoagulatien
   Cumarine;
- Antiepileptika
   Phenotoin, Phenobarbital, Primidon, Valproinsäure, Carbamazepin;
- Antihistaminika
   Chlorphenoxamin, Dimenhyrinat;
- Antimimetika;
- Antihyperttonika, Antiarryhythmika
   Lidocain, Procainamid, Chinidin, Calciumanatagonisten, Glyceroltrinitrat, Isosorbiddinitrat, Isosorbid-5-mononitrat, Pentaerythrityltetranitrat, Nifedipine, Diltiazem, Felodipin, Verapamil, Reserpin, Minoxidil, Captopril, Enlanapril, Lisinopril;
- Sympathomimetika
   Norfenefrin, Oxedrin, Midodrin, Phenylephrin, Isoprenalin, Salbutamol, Clenbutorol, Ephedrin, Tyramin, Isoprenalin, beta-Blocker wie Alprenolol, Metoprolol, Bisoprolol;
- Antidiabetika
   Biguanide, Sulfonylharnstoffe, Carbutamid, Tolbutamid, Glibenclamid, Metformin, Acarbose, Troglitazon;
- Eisenpräparationen;
- Vitamine und Vitaminoide
   beispielsweise Ascorbinsäure, Tocopherol, Tocopherolacetat, Vitamin A und Vitamin A-Derivate, Vitamin K und Vitamin K-Derivate oder Vitamin D und Vitamin D-Derivate, Riboflavin, Vitamin B12, Nicotinsäure, Nicotinsäureamid, Pyridoxin Hydrochlorid, Biotin, Folsäure, Folsäurederivate wie Tetrahydrofolsäure, 5-Methyl-tetrahydrofolsäure, 10-Formyl-tetrahydrofolsäure oder 5-Formyl-tetrahydrofolsäure;
   Carotinoide, wie z.B. beta-Carotin, Lycopin, Lutein, Astaxanthin oder Zeaxanthin; Mehrfach ungesättigte Fettsäuren, wie z.B. Linolsäure, Linolensäure, Arachidonsäure, Docohexaensäure oder Eicosapentaensäure;
   Verbindungen mit Vitamin- oder Coenzymcharakter, z.B. Carnitin, Cholinchlorid, Taurin, Kreatin, Ubichinone, S-Methylmethionin oder S-Adenosylmethionin;
- ACE-Hemmer
   Captopril, Ramipril, Enalapril;
- Anabolika;
- lod-Verbindungen;
- Röntgenkontrastmittel;
- ZNS-aktive Verbindungen;
- Antiparkinsonmittel
   Biperiden, Benzatropin, Amantadin, opioide Analgetika, Barbiturate, Bezodiazepine, Disulfiram, Lithiumsalze, Theophyllin, Valproinat, Neuroleptika;
- Zytostatika;
- Antispasmolytika;
- Vasodilatoren
   Naftidrofuryl, Pentoxifyllin.

Weiterhin können als biologisch aktive Substanzen Wirkstoffe, wie sie in der tradionellen chinesischen Medizin verwendet werden, eingesetzt werden.

Mit Hilfe des erfindungsgemäßen Verfahrens ist es möglich, wirkstoffhaltige Formulierungen durch Schmelzextrusion und Granulierung herzustellen, die eine besonders gleichmässige Form und einen niedrigen Staubanteil aufweisen. Überraschenderweise lassen sich auch Formulierungen verarbeiten, die wasserunlösliche Komponenten enthalten, ohne dass diese im Kühlmedium angelöst werden.

Die Freisetzungskurven zeigen, dass man mit Hilfe des erfindungsgemäßen Verfahrens die Freisetzungsgeschwindigkeit sicher und zuverlässig über die Partikelgröße steuern kann.

Die erfindungsgemäßen Granulate eignen sich insbesondere zur Verwendung, die Herstellung von Arzneimitteln für den Human- und Veterinärbereich sowie für Pflanzenschutzmittel, Futtermittel, Nahrungsergänzungsmittel oder diätetische Mittel.

Ebenso eignen die Granulate sich für Waschmittelformulierungen, die als biologisch aktive Substanzen Enzyme oder Aromastoffe enthalten.

Die Granulate können als solche eingesetzt werden. Weiterhin eignen sie sich als Kapselfüllungen, zur Herstellung von Trinkgranulaten oder zur Verpressung zu Tabletten.

Die Ausführung der Erfindung wird durch die folgenden Beispiele erläutert:

### Beispiele

### Einsatzstoffe:

Soluplus®, Firma BASF SE: Pfropfpolymer aus PEG 6000/N-Vinylcaprolactam/Vinylacetat, mittleres Molekulargewicht bestimmt durch Gelpermeationschromatographie 90000 -140 000 g/mol

Kollidon® VA 64, Firma BASF SE, ein Copolymer aus N-Vinylpyrrolidon und Vinylacetat im Gewichtsverhältnis 60:40, in den Pharmakopöen als "Copovidon" bezeichnet.

Kollidon® SR: co-prozessierte Mischung aus Polyvinylacetat, Polyvinylpyrrolidon K30 (Povidon nach Pharm. Eur. oder USP), Natriumlaurylsulfat und Silica im Gewichtsverhältnis 80/19/0.8/0.2
PEG 1500: Polyethylenglykol, MG 1500 g/mol
PEG 6000: Polyethylenglykol, MG 6000 g/mol

### Allgemeine Herstellvorschrift:

### Apparatur

Das zu verarbeitende Polymer wurde mittels eines gleichsinnig rotierenden Zweischneckenextruders ZSK 25 (Werner & Pfleiderer) aufgeschmolzen. Der Extruder war mit einer Dosiervorrichtung, einer Schmelzepumpe, Anfahrventil und einer Unterwassergranuliervorrichtung (GALA- LPU, Firma Gala, Xanten), versehen, weiterhin mit einer beheizten Lochplatte mit einem Bohrungsdurchmesser von 3,2 mm. Messer:5-schneiden, Drehzahl 800-2000 UPM. Die Trennung erfolgt über eine Zentrifuge (Gitter: 1.9 mm Drehzahl 1500 UPM).

Als Kühlmedium wurde gemäß den erfindungsgemäßen Beispielen 1 bis 7 ein Weissöl W118 (Fuchs Petrolub) eingesetzt, welches eine Viskosität von 16 mm²/s bei 40 °C, gemessen nach DIN 51562, aufwies.

In der nachstehenden Tabelle 1 ist die Zusammensetzung der Granulate angegeben. Die Prozentangaben beziehen sich auf Gewichtsprozent.

| Einsatzstoffe | Bsp. 1 | Bsp. 2 | Bsp. 3 | Bsp. 4 | Bsp. 5 | Bsp. 6 | Bsp. 7 |
|---|---|---|---|---|---|---|---|
| Soluplus | 100% | 90% | | | | | |
| Kollidon VA 64 | | | 95% | 90% | | 60% | 30% |
| Kollidon SR | | | | | 90% | 40% | 20% |
| PEG 1500 | | 10% | | | | | |
| PEG 6000 | | | 5% | 10% | 10% | | |
| Theophyllin | | | | | | | 50% |

In allen Fällen ließen sich gleichmäßig geformte kugelartige Granulate herstellen, die gut vom Kühlmedium abgetrennt werden konnten.

Die mittlere Partikelgröße ausgewählter Zusammensetzungen ist in der nachstehenden Tabelle 2 aufgeführt.

| Bsp. | Durchsatz | Messer | X [µm] | X [µm] | X [µm] | Span |
|---|---|---|---|---|---|---|
| Nr. | Extruder | Drehzahl | Q3=10% | Q3=50% | Q3=90% | |
| | | [UpM] | | | | |
| 1 | 5 kg/h | 1300 | 2536 | 2875 | 3138 | 0.21 |
| 2 | 5 kg/h | 1300 | 2626 | 2913 | 3355 | 0.25 |
| 4 | 5 kg/h | 1300 | 2327 | 2682 | 4124 | 0.67 |
| 6 | 5.5 kg/h | 800 | 3585 | 4109 | 4416 | 0.202 |
| 7 | 5.5kg/h | 2000 | 2663 | 3011 | 3317 | 0.217 |

Die mittlere Partikelgröße wurde mit einem Camsizer bestimmt.

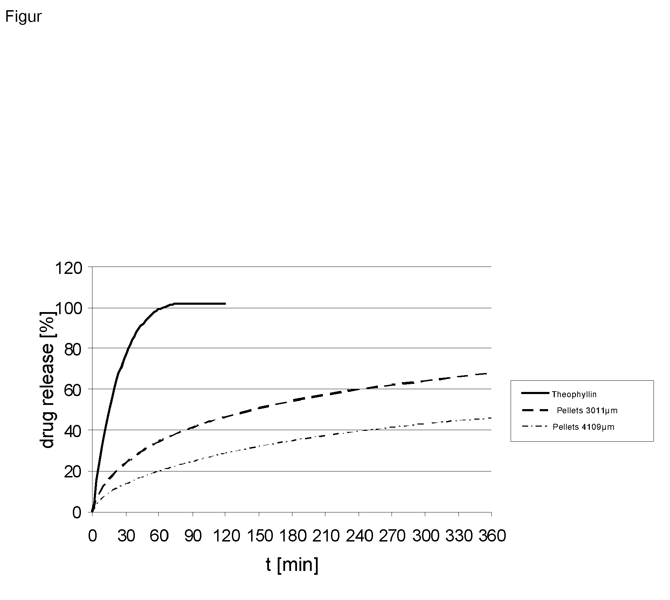

In der Figur ist ein Vergleich der Freisetzung von reinem Theophyllin (-----) mit der Freisetzung von Pellets gemäß Bsp. 6 (- - -), Durchmesser 3011 µm, und Bsp. 7 (- · - ·), Durchmesser 4109 µm, abgebildet. Die Freisetzungskurven belegen den Einfluss der Partikelgröße der Granulate auf die Freisetzung.

### Vergleichsbeispiel

Die Verarbeitung erfolgte analog Beispiel 1. Allerdings wurde ein höherviskoses Weissöl mit einer Viskosität von 20 mm²/s bei 40 °C verwendet (Winol® W20). Es kam zu einem Verkleben der Granulatperlen in der Granulationsapparatur und im Zentrifugensieb. Eine Trennung Granulat/Kühlmedium war nicht vollständig möglich.

## Patentansprüche

1. Verfahren zur Herstellung von Granulaten auf Basis mindestens eines thermoplastisch verarbeitbaren Polymeren, wobei die Granulate mindestens eine wasserlösliche Komponente enthalten, durch Schmelzextrusion und Formgebung in einem Kühlmedium, **dadurch gekennzeichnet ist, dass** als Kühlmedium ein Nichtlösemittel für das Polymer eingesetzt wird, wobei das Kühlmedium eine kinematische Viskosität von < 20 mm²/s, gemessen bei 40 °C, aufweist.

2. Verfahren nach Anspruch 1, **dadurch gekennzeichnet, dass** das Kühlmedium eine kinematische Viskosität von 10 bis 19 mm²/s, gemessen bei 40 °C, aufweist.

3. Verfahren nach Anspruch 1, **dadurch gekennzeichnet, dass** das Kühlmedium eine kinematische Viskosität von 11 bis 18 mm²/s, gemessen bei 40 °C, aufweist.

4. Verfahren nach Anspruch 1, **dadurch gekennzeichnet, dass** das Kühlmedium eine kinematische Viskosität von 12 bis 17 mm²/s, gemessen bei 40 °C, aufweist.

5. Verfahren nach einem der Ansprüche 1 bis 4, **dadurch gekennzeichnet, dass** als Kühlmedium ein Kohlenwasserstoff eingesetzt wird.

6. Verfahren nach einem der Ansprüche 1 bis 5, **dadurch gekennzeichnet, dass** als Kühlmedium ein Weißöl eingesetzt wird.

7. Verfahren nach einem der Ansprüche 1 bis 6, **dadurch gekennzeichnet, dass** das Kühlmedium temperiert wird.

8. Verfahren nach einem der Ansprüche 1 bis 7, **dadurch gekennzeichnet, dass** die Temperatur des Kühlmediums mindestens 50 °C unter der Temperatur der extrudierten Schmelze liegt.

9. Verfahren nach einem der Ansprüche 1 bis 8, **dadurch gekennzeichnet, dass** die Granulate durch Zentrifugation vom Kühlmedium abgetrennt werden.

10. Verfahren nach einem der Ansprüche 1 bis 9, **dadurch gekennzeichnet dass** die Granulate durch Filtration vom Kühlmedium abgetrennt werden.

11. Verfahren nach einem der Ansprüche 1 bis 10, **dadurch gekennzeichnet, dass** die Schmelzextrusion bei Schmelzetemperaturen von 50 bis 300 °C durchgeführt wird.

12. Verfahren nach einem der Ansprüche 1 bis 11, **dadurch gekennzeichnet, dass** als wasserlösliche Komponente ein thermoplastisch verarbeitbares Polymer eingesetzt wird.

13. Verfahren nach einem der Ansprüche 1 bis 12, **dadurch gekennzeichnet, dass** als thermoplastisch verarbeitbare Komponente eine wasserlösliche biologisch aktive Substanz eingesetzt wird.

14. Verfahren nach einem der Ansprüche 1 bis 13, **dadurch gekennzeichnet, dass** als wasserlösliche Polymere Homo- oder Copolymere von N-Vinyllactamen eingesetzt werden.

15. Verfahren nach einem der Ansprüche 1 bis 14, **dadurch gekennzeichnet, dass** als wasserlösliche Polymere Pfropfcopolymere auf Basis von Polyethern eingesetzt werden.

16. Verfahren nach einem der Ansprüche 1 bis 15, **dadurch gekennzeichnet, dass** ein Pfropfcopolymer, enthaltend Polyethereinheiten als Pfropfgrundlage und Polyvinylalkoholeinheiten als Seitenketten, eingesetzt wird.

17. Verfahren nach einem der Ansprüche 1 bis 16 , **dadurch gekennzeichnet, dass** als wasserlösliche Komponente ein Pfropfcopolymer, enthaltend Polyethereinheiten als Pfropfgrundlage und Seitenketten erhalten aus N-Vinylcaprolactam und N-Vinylacetat, eingesetzt wird.

18. Verfahren nach einem der Ansprüche 1 bis 17, **dadurch gekennzeichnet, dass** als thermoplastisch verarbeitbares Polymer Polyvinylacetat eingesetzt wird.

19. Verfahren nach einem der Ansprüche 1 bis 18, **dadurch gekennzeichnet, dass** als Polymerkomponente eine co-prozessierte Mischung enthaltend Polyvinylacetat und Polyvinylpyrrolidon eingesetzt wird.

20. Verwendung von Granulaten, erhalten durch ein Verfahren gemäß einem der Ansprüche 1 bis 19 zur Herstellung von Dosierungsformen, enthaltend eine biologisch aktive Substanz.
